# EUROPEAN PATENT APPLICATION

(11) **EP 1 074 612 A1**
(43) Date of publication of application: **07.02.2001**
(21) Application number: 99202533.8
(22) Date of filing: 04.08.1999
(51) Int. Cl.: C12N 1/20, C07K 14/135, A61K 31/70

(54) **Use of vegetal peptones for DNA vaccine production**

(71) Applicant: PASTEUR MERIEUX SERUMS ET VACCINS, 69007 Lyon (FR)
(72) Inventor: Speck, Denis, 69110 Sainte Foy les Lyon (FR)
(74) Representative: Gros, Florent

(57) **Abstract**

A process for producing a DNA vaccine in which a non-infectious DNA is multiplied in a microorganism host by cultivation in a suitable medium and the DNA is purified and further formulated in a DNA vaccine composition, characterized in that the media comprises only vegetal peptones as the substantial nitrogen source.

## Description

The invention relates to the use of vegetal peptones for the cultivation of micro-organisms containing DNA vectors which are intended to be further purified and used for DNA vaccination purposes.

### State of the art

Media used in microbial processes can be grouped into 2 general types: defined and complex. Defined media are those composed of chemically defined compounds. These media allow the clear definition of the limiting factor and are very useful for physiological studies. A medium composed of glucose, an ammonium compound, mineral salts, trace elements and pure vitamins is an example of a defined medium. The addition of a peptone or yeast extract to this medium makes it a complex. Complex media are generally used for production purposes.

Luria Broth (LB) is one of the most widely used complex media for low scale bacterial culture. LB2XR, a reformulated medium (twice concentrated) based on the LB composition, is currently used in the development laboratory for production of plasmid DNA. The nitrogen source of this medium is tryptone, a trypsin enzymatic digest of casein, which is a protein from milk.

Nitrogen requirements for bacterial growth can also be met by inorganic or organic sources. The majority of micro-organisms utilizes ammonia as a nitrogen source, generally supplied as (NH₄)₂SO₄, which is the preferred source used both in laboratory and in industry for defined media.

The use of media containing substantially as nitrogen source vegetal hydrosylates has not been much described in the literature. This has been described for amino acid production with transformed micro-organisms (see US4442208 of Ajinomoto and US 4444885 of Kyowa H).

Also W09854296 from Chiron describes a medium for culturing pathogenic bacteria to produce an immunogenic polysaccharide, said medium comprising non-animal derived proteinaeous material.

The objective of the present invention is to produce a DNA vaccine of good quality, imunogenicity and safety.

### Summary of the invention

To this end, the invention related to a process for producing a DNA vaccine in which a non-infectious DNA is multiplied in a microorganism host by cultivation in a suitable medium and the DNA is purified and further formulated in a DNA vaccine composition, characterized in that the media comprises only vegetal peptones as the substantial nitrogen source.

### Detailed description of the invention

The present invention relates to a process for producing a DNA vaccine in which a non-infectious DNA which encodes for an immunogenic factor is multiplied in a microorganism host. The immunogenic factor of the present invention may come from any pathogenic virus bacteria, superior cell, pluricellular parasites. This includes bacteria leading to infections selected from *meningitidis, diphteria,* leading to infectious, *tetanus,* whooping cough or ulcers for instance. Accordingly, preferred pathogenic bacteria include *Helicobacter pylori, Haemophilus influenzea, Corynebacterion diphtheria, Neisseria meningitidis, Bordetella Pertussis* and *Clostrium tetanis.* The virus immunogenic factor can originate from the cytomegalovirus, HIV, hepatitis A, B or C, influenza, etc. Superior cells *include plasmodium falciparum, schigellose,* ect.

The term "immunogenic factor" as used herein, means any factor that is capable of stimulating the immune system of a human or animal. Such immunogenic factors include antigenic proteins and especially virulence factors and fragments thereof. Virulence factors are defined as being associated with the virulence of a bacteria and include such factors as the vaculating cytotoxin VacA produced by *Helicobacter pylori.* Other virulent factors are described by Rappuoli, R. et al., European Journal of Gastroenterology and Hepatology of *Helicobacter pylori* infection. Proceedings of an interdisciplinary meeting (Genova, June 18-19, 1993) J.J. Misiewicz, Ed. (CS Current Science), pp S76-S78 (incorporated herein by reference).

This immunogenic factor is, in humans or animals, encoded by a non-infectious DNA. This DNA may be in the form of a plasmid, phagemid or other virus derivative vectors, or also integrated in the genome of the host.

This non-infectious DNA is multiplied by cultivating the host in a suitable medium. Any standard medium for cultivating bacteria may be used as the basis for the medium of the present invention, provided that the medium does not contain animal derived proteinaceous material, but substantially only peptones of vegetal origin.

Preferably, the medium of the present invention comprises a carbon and an energy source, a nitrogen source, essential salts and optionally a selecting agent, such as an antibiotic, for selecting the micro-organisms to be cultured.

The medium of the present invention may be a solid or liquid medium. Examples of standard liquid media which may form the basis of the medium of the present invention include Brucella Broth (without tryptone and peptamine), Waston medium (without casamino acids), Mueller Miller medium (without heart infusion and casein hydrolysate), CL Medium (without casamino acids) and Franz A Medium. Standard solid media can be prepared from any of the liquid media by the addition of a solidifying agent such as agar.

The term "cultivating" as used herein means the maintenance of, and preferably the growth of the host. Host growth is herein defined as an increase in host.

The media thus comprises substantially as nitrogen source only vegetal peptones from soy beans, cotton seeds, potatoes, wheat gluten, rice seeds, maize seeds, malt seeds, etc, for example. As an example no intended to be a limitation, soy bean derived protein compositions can be prepared by enzymatic digestion of soy bean meal or soy isolate using standard enzymes such as papain. Soy bean derived protein compositions can also be obtained by acid hydrolysis of a soy isolate. Other vegetal derived proteinaceous materials can be obtained by either enzymatic digestion or by acid hydrolysis.

The vegetal peptones should preferably lead to the following criteria: 1) growth capacity: the peptone should be able to support specific growth rate and final biomass density as high as with tryptone and to lead to equivalent final biomass density; 2) solubility : the peptone should be easy to dissolve in order to simplify the medium preparation protocol. Also, it could be used in batch cultures at a high initial level in the medium or in feeding solution for a fed-batch cultivation and therefore should be soluble even at high concentrations. 3) Resistance to heat treatment: the peptone should be stable at high temperatures for *in situ* sterilization process. The medium should not be altered during this process.

Yeast extacts may be also used to supplement the media.

Suitable culture conditions for the production of DNA, including the duration of the culture, will vary depending on the bacteria being cultured. However, one skilled in the art can easily determine the culture conditions required for the production of the DNA by following standard protocols, such as those described in the series Methods in Microbiology, Academic Press Inc., (incorporated herein by reference) and, if necessary by performing a number of standard experiments to determine suitable culture conditions.

The DNA can be isolated from the bacterial culture using a number of standard techniques including those described by Sambrook *et al.,* Molecular Cloning, A Laboratory Manual, Cold Spring Harbor Laboratory Press, U.S.A., 1989, incorporated herein by reference.

Formulation of the DNA vaccine composition may require the use of stabilisers, such as those described in EP353108 or EP869814, and the use of suitable adjuvants such as aluminium salts, phosphates and hydroxydes ; Freund adjuvant; N-acétylmuramyl-L-alanyl-D-isoglutamyl-L-alanine-2-[1,2-dipalmitoyl-sn-glycéro-3-(hydroxyphosphoryloxy)] (see Sanchez-Pescador *et al., J.* Immu., 141, 1720-1727 1988); molecules derived from *Quillaja saponaria,* as the Stimulon® (Aquila, US) the Iscoms® adjuvant (CSL ltd, US) ; all molecules from cholesterol or analogy thereof, as the DC-Chol® (Targeted Genetics) ; the glycolipid Bay R1005® (Bayers DE) ; antigens from *Leishmania brasiliensis* as the LeIF (technical name) available a Corixa Corp. (US); polymers from the polyphosphazenes family, as the Adjumer (technical name) available at the "Virus Research Institute " (US).

The DNA can be formulated in liposomes or can be coupled to fatty acids derivatives, including cholesterol molecules.

The vaccines prepared by the process of the present invention can be used to vaccinate an individual against a bacterial viral or parasite infection. The vaccines prepared by the process of the present invention may be provided as a pharmaceutical composition comprising the vaccine of the present invention in admixture with a pharmaceutically acceptable carrier and adjuvants as mentioned above.

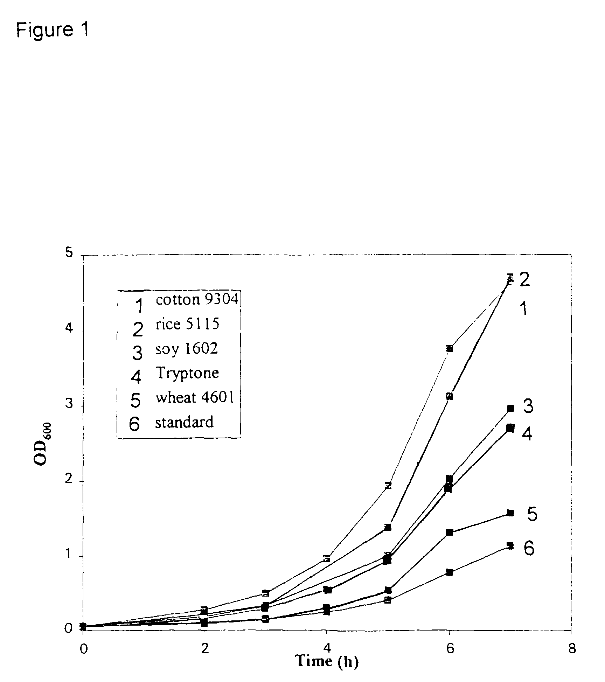

The vaccines prepared by the process of the present invention can be administered by oral or parenteral route, including intravenous, intramuscular, intraperitoneal, subcutaneous, transdermal, airway (aerosol) rectal and topical administration
Figure 1 displays the result of the effect of vegetal nitrogen sources on growth in shake-flask cultures in a medium M3 containing 20 g/l of cotton peptone 9304 and 5 g/l of yeast extract. Optical density at 600 nm is plotted as a function of cultivation time

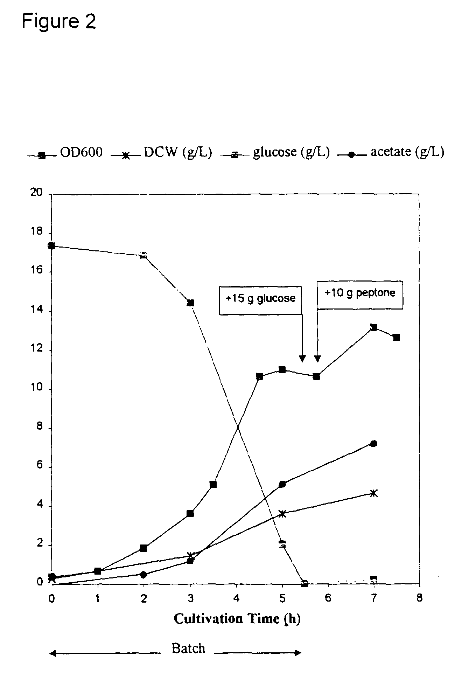

Figure 2 displays the result of a batch culture of *Escherichia coli* DH10B containing plasmid VR2402 in a medium M3 containing 20 g/l of cotton peptone 9304, 5 g/l of yeast extract and 20 g/l of glucose. Optical density at 600 nm, biomass concentration (DCW), glucose concentration and acetate concentration are plotted as a function of cultivation time. Arrows indicate the addition of 15 g of glucose (t= 5.5h) and 10 g of cotton 9304 peptone (t=5.75h) to the culture.

### Materials and methods used in the following examples

Strain and plasmid: *Escherichia coli* strain DH10B (Gibco BRL, Life Technology, Inc. Gaithersburg, MD, USA) harbouring plasmid VR-2402 was taken from the master cell bank *Escherichia coli* strain DH10B is often used for the production of plasmid DNA (Horn et al, 1995, Chen et al, 1997) especially because of the *endA* mutation that provides high-quality plasmid DNA preparations.

Plasmid VR-2402 (Vical, Inc. San Diego, CA, USA) is about 7.4 kb long and contains the coding sequence for the Respiratory Syncytium Virus (RSV) F glycoprotein. It was designed for gene therapy and DNA production processes. Indeed, the VR-2402 plasmid backbone is derived from the pUC18 plasmid which is a high copy number plasmid (Minton et al, 1988, Lin-Chao et al, 1992) and allows high-yield plasmid product fermentation. It also contains the selectable marker for kanamycin resistance that is more appropriate than ampicillin for fermentation scale-up. Finally, the combination of human cytomegalovirus (CMV) strong promoter/enhancers with bovine growth hormone (BGH) polyadenylation termination sequence is used to obtain high level expression after injection in many cell types.

Raw materials: the different protein hydrolysates of vegetal origin used in this study were kindly supplied as free samples by the following manufactures: Organotechnie, La Courneuve, France and Quest International, Maarssen, Netherlands. Table 1 summarises the main characteristics of these raw materials. DH is estimated from the ratio between the alpha amino nitrogen (AN) and the total nitrogen (TN).

Tryptone and yeast extract were supplied by Difco Laboratories, Detroit, MI, USA.

**Table 1 :**

| Protein hydrolysates from vegetal raw materials and their main characteristics (DH=Degree of Hydrolysis, NA=Not available) | | | | |
|---|---|---|---|---|
| Product | Description | Solubility | DH | Supplier |
| Soy peptone A1 | Soy protein enzymatic digest | 50 g/l | 16% | Organotechnie |
| Soy peptone A2 | Soy protein enzymatic digest | 50 g/l | 25% | Organotechnie |
| Soy peptone A3 | Soy protein enzymatic digest | 50g/l | 29% | Organotechnie |
| Wheat peptone E1 | Wheat protein enzymatic digest | 50g/l | 20% | Organotechnie |
| Malt extract R2 | Wheat gluten enzymatic digest | 30g/l | NA | Organotechnie |
| Hypep Dev 4402 | Wheat gluten enzymatic digest | 300 g/l | 8% | Quest |
| Hypep Dev 5115 | Rice protein enzymatic digest | 300 g/l | 17.1% | Quest |
| Hypep Dev 4601 | Wheat gluten enzymatic digest | 300 g/l | 14.9% | Quest |
| Hypep Dev 1602 | Soy protein enzymatic digest | 300 g/l | 22% | Quest |
| Hypep Dev 9304 | Cotton protein enzymatic digest | 300 g/l | 18.6% | Quest |
| Amisoy | Soy protein acid digest | <25g/m | 69-74.6% | Quest |

Glycerol stock: a working cell bank (WCB) was prepared from the MCB SGA 1/10/96 in order to inoculate all cultures with bacteria grown in the same conditions. The WCB consisted in a 1% glycerol stock culture grown in Luria Broth 2X concentrated (LB2X) medium containing 25µg.mL⁻¹ kanamycin (Sigma Chemical Co., St Louis, MO, USA). The cells were grown until they reached an optical density at 600nm (OD₆₀₀) of 3 Units and then centrifuged at 5000g, 4°C, 15 min. The bacterial pellet was resuspended in a chilled solution made up with 50% (v/v) LB2X and 50% (v/v) freezing mix solution (2.46g.L⁻¹ MgSO4, 5g.L⁻¹, NaCl, 200g.L⁻¹ 100% glycerol). It was divided up into 1ml samples and maintained at-70°C.

Media: LB2X medium composition is given in Table 2. This medium served as a standard. Composition of the M3-bases culture media, a significant part of this study, is described in the examples section. Mineral salts, trace elements solution, kanamycin, and glucose are sterilized separately by filtration on a 0.22µm filter (Corning Costar Corp., Cambridge, MA, USA).

**Table 2**

| Standard medium LB2X | | |
|---|---|---|
| Components | Amount | Unit |
| Tryptone | 20 | g.L⁻¹ |
| Yeast extract | 10 | g.L⁻¹ |
| NaCI | 10 | g.L⁻¹ |
| MgCl₂ 1M^{a} | 2 | mL.L⁻¹ |
| Tris 2M pH7,5^{a} | 10 | mL.L⁻¹ |

| | | |
|---|---|---|
| a These components were sterilized separately by filtration | | |

For shake flask culture experiments, stock solutions were prepared in order to minimise problems of precipitation due to the large amount of phosphates needed to maintain a correct pH during the cultivation. A 10X stock solution containing 250g.L⁻¹ Na₂HPO₄, 19 g.L⁻¹ KH₂PO₄, and 3ml.L⁻¹ trace elements solution was prepared. Concentrations of protein hydrolysate and yeast stocks varied according to the experiment objective.

**Table 3.**

| Media composition for shake-flask cultures | | |
|---|---|---|
| Components | Amount | Unit |
| Na₂HPO₄7H₂O | 25 | g.L⁻¹ |
| KH₂PO₄ | 1.9 | g.L⁻¹ |
| Trace elements solution ^{a,}^{b} | 0.3 | mL.L⁻¹ |
| Nitrogen source | variable | g.L⁻¹ |
| Yeast extract | variable | g.L⁻¹ |
| MgSO₄7H₂O^{a} | 400 | mg.L⁻¹ |
| CaCl₂.2H₂O^{a} | 11 | mg.L⁻¹ |
| FeCl₃6H20^{a} | 7 | mg.L⁻¹ |
| Kanamycin^{a} | 25 | mg.L⁻¹ |

| | | |
|---|---|---|
| ^{a} These components were sterilized separately by filtration | | |
| ^{b} Trace elements solution contained per liter 10 g AlCl₃.6H₂O, 4 g CoCl2.6H20, 1 g CuCl₂.2H₂O, 5g H₃BO₃, 10 g Kl, 10 g MnSO₄.6H₂O, 2 g Na₂MoO₄.2H₂O, 0.45 g NiSO₄6H₂O, 2g ZnSO_{4.}7H₂O, and 162.2 g K₃C₆H₅O₇ | | |

For bioreactor experiments, HK₂PO₄, trace elements solution, protein hydrolysate and yeast extract were dissolved together in UF water and autoclaved in the culture vessel for 30 min at 121°C. Before inoculation, mineral salts, kanamycin, and initial glucose were directly added into the culture vessel by sticking a sterile needle equipped with a 0,22µm filter through a plasma membrane.

### Shake-flask experiments

Erlenmeyer flasks with four baffles were used for shaken cultivations. 50-mL medium in 500mL flasks with 0.5 or 1 mL glycerol stock as inoculum were cultivated at 37°C and 170rpm for 7 hours in an orbital shaker (Model 4581, Forma Scientific, Inc. Marietta, OH, USA). A culture sample was taken every two hours along the cultivation process to determine the OD₆₀₀ and after 7 Hours, both OD₆₀₀ and dry cell weight (DCW) were measured.

### Bioreactor experiments

A 2-L total volume bioreactor (Biolafitte, Maisons-Laffite, France) was used. It was equipped with a control cabinet which regulates DO, pH, and antifoam additions (Figure 1). Temperature was maintained at 37°C with an additional water bath that delivers water of the preadjusted temperature to the double wall of the culture vessel. pH was maintained at 7.00 by either adding 2M H₂SO₄ or 4N NaOH with peristaltic pumps. A steam sterilizable pH probe model InPro 3000 (Mettler-Toledo,Paris, France) was used to measure the culture pH. The DO was maintained at 30% of air saturation by a three-step cascade of regulation: (1) manual increase of the stirrer speed until 1000 rpm then (2) opening of the airflow valve regulated by the control cabinet and (3) adding of pure oxygen to air supplied in the sparger ring. Air and O₂ flow rates were controlled with flowmeters. DO was measured with an Ingold Electrodes (Mettler Toledo) steam sterilizable polarographic electrode. Foam was depressed by controlled supply of 2% (v/v) Struktol J673. In fed-batch experiments, feed was supplied by a peristaltic pump (Model 505-Du, Watson-Marlow Limited, Falmouth, UK) using a double silicone element (1.66mm bore-2.4mm wall).

After the sterilisation step, the DO electrode was calibrated in the final medium at 37°C by gazing the medium either with nitrogen or with air to set the 0% and the 100% respectively. A 10% (v/v) working volume inoculum grown overnight for 15 hours (see shake-flask experiments for growth conditions) was aseptically transferred to the culture vessel. The initial culture conditions were as follows: initial culture volume=1.5L for batch- and 1L for fed-batch cultures, stirrer speed=450 rpm, temperature=37°C, pH=7.00.

Analytical Procedures - Biomasse concentration: the cell density was determined by measuring the OD₆₀₀ with a UV/VIS spectrophotometer (Model V-530, Jasco Corp., Tokyo, Japan). Culture samples were diluted with UF water to obtain an OD₆₀₀ between 0.2 and 0.7.The dry cell weight (DCW) was determined by centrifuging in borosilicate glass tubes two culture samples of 5ml at 4000 tr/min, 4°C in C60 rotor for 10 min (Jouan model KR4-22, Saint Herblain, France). The pellets were then resuspended in UF water before centrifuging again. The supernatant was removed, and the pellet was dried in an oven at 105°C for 24 hours. After being cooled for 1 hour, the dried cells were weighed on a precision balance (Sartorius MC210P, Goettingen, Germany).

Analytical Procedures - Glucose and acetate concentration: at first, glucose concentration was determined using an enzymatic kit from Boehringer Mannheim Biochemicals (Indianapolis, IN, USA). For the last part of the study, glucose concentration was determined using a clinical glucose analyser (Model Glucose Analyser 2, Beckman Instruments, Inc. Fullerton, CA, USA. After the instrument was calibrated with 1.5 g.L⁻¹ glucose standard solution, appropriately diluted experimental samples were analyzed according to the manufacturer's instructions. Acetate concentration was determined using an enzymatic kit from Boehringer Mannheim Biochemical (Indianapolis, IN, USA).

Analytical Procedures - plasmid vialibility: viability was measured by direct plating onto non selective LB agar (BioMérieux, Marcy l'Etoile, France). The sterile culture sample was diluted with 0.9% sodium chloride solution to yield 30-300 colonies per plate and incubated at 37°C for 24 h. Viability was calculated by taking the average number of colonies from three plates.

Analytical Procedures - plasmid stability: plasmid stability was measured by transferring 100 colonies from a non selective LB agar plate onto selective (25 mg.L⁻¹ kanamycin) LB agar plates.

Analytical Procedures - Plasmid DNA analysis: during the cultivation process, several culture samples corresponding to different values of cell density were centrifuged in 1 ml eppendorf tubes for 10 min at 4°C, 4500 tr/min (Prolabo Model SR2002, Paris, France). The supernatant was removed and the pellet was stored at -20°C for further DNA analysis. Plasmid DNA was purified according to protocol of either QIAGEN Plasmid Mini Kit, or QlAfilter Plasmid Midi kit (Qiagen GmbH, Hilden, Germany). This protocol is based on a modified alkaline lysis procedure, followed by binding of plasmid DNA to QIAGEN Anion Exchange Resin under appropriate low salt and pH conditions. RNA, proteins, dyes, and low molecular weight impurities are removed by a medium salt wash. Plasmid is eluted in a high salt buffer, and concentrated and desalted by isopropanol precipitation.

The amount of purified plasmid DNA was estimated by measuring the OD at 260nm (OD₂₆₀) of a sample diluted at 1% (v/v) with UF water. A OD₂₆₀ unit represents a plasmid DNA concentration of 50*g/L. OD at 280nm (OD₂₈₀) was also measured in order to calculate the ratio OD₂₆₀/OD₂₈₀ which reflects the purity of the preparation with regards to protein.

In order to check the plasmid integrity and purity, the supercoiled DNA along with others DNAs in the sample were loaded onto and separated on a 1% agarose gel containing 1*g.ml⁻¹ ethidium bromide (EtBr) run for 75 min at 0.1A. The bands of DNA were then visualized with a UV-lamp and a picture was taken using a MP4 camera (Polaroïd, Cambridge, MA, USA).

The following examples are presented for illustrative purposes only.

### Example 1 - Selection of a vegetal protein hydrolysate

Growth experiments were conducted in shake flask cultures with a M3-bases medium (Table 3 above). In order to evaluate the experimental error on the analysis measurements during growth cultivation, a shake flask experiment was repeated six times exactly in the same conditions with a M3 based medium containing the same amount of complex components as in LB2X standard medium

Plackett and Burman experimental matrix design (1946) was used to study the influence of each vegetal peptone out of the eleven to be tested on *E.coli* DH10B growth. This methodology gives mathematical models and their statistical significance, which are useful for studying the effects of the selected variables on different growth parameters.

The nitrogen supply was set at 0.5 g.L⁻¹ by considering the fact that the yield coefficient for nitrogen is equal to 6 and that the medium we used was designed for 3 g.L⁻¹ DCW. The medium could also contained 1 g.L⁻¹ yeast extract.

4 growth parameters were measured: optical density at 600 nm (OD₆₀₀) after 7 hours of growth; dry cell weight (DCW, g.L⁻¹) after 7 hours of growt; maximum specific growth rate (µₘₐₓ, h⁻¹); plasmid DNA yield (Y_{p/x}, mgDNA. g⁻¹ DCW).

According to this methodology, five peptones were selected for their positive influence on growth according to the two corresponding diagrams: malt (R2), rice (5115), cotton (9304), wheat (E1), and soy (A1).

### Example 2 - Screening by testing peptones separately

Further experiments were conducted in the same conditions as in the example 1, *i.e.* medium for shake cultures (Table 3) supplemented with 1g.L⁻¹ yeast extract and a single peptone at a concentration equivalent to a supply of 0.5 g nitrogen per liter. The five peptones selected from example 1 results were tested. Soy 1602, which was supposed to have the lowest effect on growth, was also tested as a negative control, and tryptone as a reference.

**Table 4**

| Summary of the results obtained for the screening of a vegetal peptone. | | | | | |
|---|---|---|---|---|---|
| Product | Origin | Supplier | Solubility (g.L) | Growth Capacity | Visual aspect after heat treatment |
| Soy peptone A1 | Soy | Organotechnie | 50 | ++ | Clear |
| Wheat peptone | Wheat | Organotechnie | 50 | ++ | Clear |
| Malt extract R2 | Malt | Organotechnie | 30 | ++ | Clear |
| Hypep Dev 4402 | Wheat | Quest | 300 | NT^{a} | Cake |
| Hypep Dev 5115 | Rice | Quest | 300 | +++ | Cloudy |
| Hypep Dev 4601 | Wheat | Quest | 300 | + | Precipitate |
| Hypep Dev 1602 | Soy | Quest | 300 | ++ | Cloudy |
| Hypep Dev 9304 | Cotton | Quest | 300 | +++ | Clear |
| Amisoy | Soy | Quest | <25 | NT^{a} | Precipitate |
| Tryptone | Bovine | Difco | Na^{b} | ++ | Clear |

Rice 5115 and cotton 9304 gave the best results regarding the final biomass whereas malt R2, which was expected to have the highest effect on growth gave equivalent results as soy 1602, the negative control, soy A1, wheat E1, and tryptone.

Results are shown in Figure 1. The high capacity of rice 5115 and cotton 9304 to support growth was confirmed. Better results were obtained with every tested vegetal peptone than with standard medium in terms of biomass obtained for a defined length of culture. The final dry cell mass after 7 hours was 3.6 times higher in M3 medium supplemented with cotton 9304 than in LB2X medium.

Global results for the screening of vegetal peptones, in terms of all selection criteria, are summed up in table 4 above: both rice 5115 and cotton 9304 are good candidates among Quest vegetal peptones. Nevertheless, after heat treatment (30 min at 121°C), a thin cake appeared in rice 5115 peptone stock solutions (50g.L⁻¹) whereas cotton 9304 stock solutions remained clear. The vegetal peptone Hypep Dev 9304, an enzymatic digest of cottonseed protein, was thus the best candidate among eleven tested vegetal peptones.

### Example 3 - High cell density culture in batch experiment

The next objective was to achieve high cell density culture of E. *coli* DH10B using the selected vegetal peptone Hypep Dev 9304. A sequential approach was used. At first, batch experiments were performed to characterize the strain behaviour in the new medium, and changes were conducted on each new culture in order to improve the latest results. Finally, a fed-batch process was defined to overcome the encountered problems.

The M3 medium designed to support a final cell density of 10 g DCW.L⁻¹ was supplemented with 20 g.L⁻¹ cotton 9304 peptone (same concentration as tryptone in standard LB2X medium) and 5 g.L⁻¹ yeast extract. The medium composition is described in Table 5 below. The initial glucose level was set to 20g.L⁻¹. The following results are based on several batch experiments. Figure 2 shows the results of a representative batch experiment.

In order to determine if some nutrient other than glucose was limiting cell growth, different components of the medium were added to the culture vessel when growth stopped. The addition of the limiting nutrient should induce a sudden decrease of the DO. Pulses of each mineral salt and trace elements had no significant influence on the DO. We proceeded therefore to a pulse of cotton 9304 peptone. Approximately 15 g were added. The DO did not show immediate variations but one hour later, the cell density had slightly increased (2.5 OD₆₀₀ units).

Further experiments showed that it is important to increase peptone concentration in the medium so as to support a final cell density of 20 g DCW.L⁻¹. We need to lower the acetate production by growing cells in glucose limiting conditions in a fed-batch process. Cells must be grown at a lower growth rate than that the critical growth rate for acetate formation.

**Table 5**

| Media composition for bioreactor cultures | | | | |
|---|---|---|---|---|
| Components | Amount | | | Unit |
| | Batch | Fed-Batch | | |
| | | Initial medium | Feeding | |
| KH₂PO₄ | 1.1 | 2.2 | -- | g. L⁻¹ |
| Trace elements solution ^{a}, ^{b} | 1 | 2 | -- | mL.L⁻¹ |
| Hypep Dev 9304 | 20 | 70 | -- | g.L⁻¹ |
| Yeast extract | 5 | 10 | -- | g.L⁻¹ |
| MgSO₄.7H₂O ^{a} | 400 | 800 | -- | Mg.L⁻¹ |
| CaCl₂.2H₂O ^{a} | 11 | 22 | -- | Mg.L⁻¹ |
| FeCl₃6H₂O ^{a} | 7 | 14 | -- | Mg.L⁻¹ |
| Kanamycin ^{a} | 25 | 25 | -- | Mg.L⁻¹ |
| Glucose ^{a} | 20 | 5 | 300 | g.L⁻¹ |

| | | | | |
|---|---|---|---|---|
| ^{a} These components were sterilized separately by filtration | | | | |
| ^{b} Trace elements solution contained per liter 10 g AlCl₃.6H₂O, 4 g CoCl2.6H20, 1 g CuCl₂.2H₂O, 5g H₃BO₃, 10 g Kl, 10 g MnSO₄.6H₂O, 2 g Na₂MoO₄.2H₂O, 0.45 g NiSO_{4.}6H₂O, 2g ZnSO_{4.}7H₂O, and 162.2 g K₃C₆H₅O₇ | | | | |

Also, it is important to regulate the DO level so as to maintain strict aerobic conditions and therefore avoid anaerobic fermentation and by-products formation such as lactic, oxaloacetic, pyruvic and propionic acids.

### Example 4 - High cell density culture in fed-batch experiments

A new medium was designed to support a final cell density of 20 g DCW.L⁻¹. Considering the batch experiments results in Example 3, the cotton 9304 peptone yield coefficient was estimated at 0.275 as about 5.5 g DCW were obtained with 20 g peptone. Therefore, the peptone concentration was increased to 70 g.L⁻¹ to reach a final cell density of 20 g DCW.L⁻¹. As the previous medium was designed for 10 g DCW.L⁻¹, the concentration of every other component was multiplied by Example 5 - Plasmid DNA production

Plasmid stability was determined for each sample of examples 1-4. One hundred percent of the cells contained the plasmid as determined by direct plating onto selective and non selective plates which means that the plasmid is stable.
Plasmid DNA was purified using a kit. An approximate yield was obtained from several purification assays that were performed on samples taken from shake-flask, batch and fed-batch experiments described in the above examples. An estimation of the plasmid DNA yield that could be expected from a non-induced process involving vegetal peptone was about 4 mg plasmid DNA per g of biomass. Similar plasmid DNA yields have been obtained previously with non-induced LB2X grown cultures but a temperature switch to 42°C allowed to reach plasmid DNA yields of about 10 mg.g⁻¹ DCW in this medium.

## Claims

1. A process for producing a DNA vaccine in which a non-infectious DNA is multiplied in a microorganism host by cultivation in a suitable medium and the DNA is purified and further formulated in a DNA vaccine composition, characterized in that the media comprises only vegetal peptones as the substantial nitrogen source.

2. The process as claimed in claim 1, wherein the vegetal peptones are selected from the group of soya, cotton, wheat gluten, rice and malt.

3. A process as claimed in claim 1, wherein the host microorganism is a prokaryotic cell.

4. A process as claimed in claim 1, wherein the DNA vaccine encodes an immunogenic factor that is capable of stimulating the immune system of a human or animal.

5. A process as claimed in claim 3, wherein the DNA is under the form of a plasmid, phagemid or integrated in the genome of the host.

6. A non-infectious vaccine composition obtained from the process of claims 1 to 5 comprising a non-infectious DNA encoding an immunogenic factor said composition being substantially free of any peptones traces of animal origin.
